Europäisches Patentamt

European Patent Office

Office européen des brevets

(19)

(11) Publication number: **0 147 920**
**B1**

(12) **EUROPEAN PATENT SPECIFICATION**

(45) Date of publication of patent specification: **23.12.87**

(51) Int. Cl.⁴: **A 61 B 17/08**

(21) Application number: **84306465.0**

(22) Date of filing: **21.09.84**

(54) **Method and instrument for applying a fastener to a tissue using means to grasp, guide and pull the fastener through the tissue.**

(30) Priority: **23.09.83 US 535110**

(43) Date of publication of application:
**10.07.85 Bulletin 85/28**

(45) Publication of the grant of the patent:
**23.12.87 Bulletin 87/52**

(84) Designated Contracting States:
**BE DE FR GB IT NL SE**

(56) References cited:
**FR-A-2 322 578**

(73) Proprietor: **ETHICON INC.**
**U.S. Route 22**
**Somerville New Jersey 08876 (US)**

(72) Inventor: **Barkley, Andrew**
**93 Galloping Hill Road**
**Basking Ridge New Jersey 07920 (US)**

(74) Representative: **Jones, Alan John et al**
**CARPMAELS & RANSFORD**
**43 Bloomsbury Square**
**London, WC1A 2RA (GB)**

Courier Press, Leamington Spa, England.

## Description

This invention relates, in general, to an instrument for fastening together portions of tissue in surgical procedures.

In various surgical procedures fasteners in the form of staples, and the like, are employed for holding tissue portions together to facilitate healing of a wound or incision. For example, a locking staple having a tongue and groove structure by which the staple is locked is disclosed in US—A—2,881,762. A metal staple, especially adapted for ligating blood vessels, is disclosed in US—A—3,079,608. International Patent Application No. PCT/SU79/00049 discloses a variety of fastening devices and instruments for performing circular anastomosis on the large intestine. The aforementioned disclosures serve as examples for a wide variety of tissue fastening devices and techniques that may be employed in general and/or specific surgical situations.

One common type of fastening device for joining or holding together soft tissue portions is the generally U-shaped staple which is typically fabricated from suitable metals. Such staples, although generally described as having two legs joined by a link to define a U-shape when unclinched, may also be regarded as having a configuration of an open loop when unclinched. The legs need not be necessarily parallel but are typically adapted for penetrating the tissue portions and for receiving between them some of the tissue material.

Other examples of U-shaped or open loop staples as well as methods and instruments for applying said staples to tissue are disclosed in US—A—3,252,643; US—A—3,482,428; US—A—3,692,224; US—A—3,790,057; US—A—3,795,034; US—A—3,889,683; US—A—4,198,982; US—A—4,316,468; and US—A—4,329,576.

Other tissue fastening devices have been proposed and differ from staples per se in that these other devices may have a plurality of components that do not have to be clinched in the manner of a metal staple. One such device is disclosed in US—A—4,060,089 and includes a fastener strip provided with a plurality of longitudinally spaced parallel prongs which are adapted to penetrate overlapped tissue portions from one side so that the distal ends of the prongs project from the other side of the tissue portions.

The fastener device further includes a retainer strip which is adapted to be placed on the other side of the tissue portions opposite the fastener strip to engage the ends of the projecting fastener strip prongs and to secure the tissue portions tightly between the fastener strip and the retainer strip. The fastener strip prongs each include a plurality of spaced apart engaging members for engaging the retainer strip at the desired position relative to the prongs. This provides for the capability of adjusting the distance between the fastener strip and the retainer strip. Such a fastening device may be fabricated from a bio-degradable or absorbable material.

Other patents such as US—A—2,286,578 and US—A—3,638,654 disclose instruments for applying flexible sutures with needles that are inserted into the tissue portions.

US—A—4,006,747 discloses the application of a flexible fastener to tissue by means of a slotted hollow straight needle with a plunger for pushing the fastener through the needle. The application of a similar fastener in a non-surgical situation is disclosed in US—A—4,215,807.

Also, in European patent application No. 84304134.4, (EP—A—0 129 442), an instrument having a pair of spaced apart needles oriented in generally parallel planes is disclosed. Each needle has a distal end adapted for piercing the tissue portions. Each needle is hollow and has a passage extending along its length from a fastening member receiving opening to a discharge opening at the distal end of the needle. Each needle also has a slot extending along its length in communication with the passage and facing the slot of the other needle. According to the method of that patent application, the fastener is applied by first approximating tissue portions in generally face-to-face relationship. The hollow needles are inserted through the approximated tissue portions to locate the receiving openings on one side of the wound or incision and to locate at least portions of the discharge openings on the other side of the wound or incision. The fastening member is oriented with each of the legs disposed in one of the needle passages and with the link extending through the needle slots between the needles. The fastening member is urged along the needles to locate at least a portion of the link on one side of the incision adjacent to one of the tissue portions and locate a portion of each leg on the other side of the incision where it is restrained by a suitable receiver. The needles are withdrawn and the fastener remains holding the tissue together.

Although many of the above discussed types of tissue fastening devices and techniques are satisfactory in various applications, there is a need to provide an improved instrument for fastening mammalian tissue with reduced trauma. It would also be desirable to provide an improved instrument for use with fasteners fabricated from absorbable materials that can provide primary approximation of the tissue edges to insure that the tissue edges will not lose contact. The instrument should insure that the staple legs, as they pass through the tissue, fully align with the openings in the receiver. The instrument should eliminate the possibility of misalignment of the staple legs with the openings in the receiver. When misalignment occurs, blind movement of the legs is required until an opening is met, very often causing undue trauma to the tissue.

Also, it would be desirable for the instrument to be relatively simple yet effective and rapidly operating for applying a variety of fasteners.

The present invention provides an instrument as defined in claim 1 for applying a fastener or a group of fasteners to close a wound or incision in mammalian tissue by holding together portions of the tissue defining the wound or incision so as to facilitate healing of the wound or incision. The instrument can be employed with a variety of types of fasteners comprising at least a fastening member having at least one leg and suitable stop means to prevent that leg from totally passing through the tissue. A preferred fastening member has a pair of legs joined by a suitable link with that link lying against one of the tissue portions on one side of the wound and acting as the stop means. The fastener also includes a receiver having at least one opening which is placed on the opposite side of the wound and retains the leg penetrating the tissue.

The instrument preferably has a needle which, at its distal end, has means for guiding and/or grasping the leg of the fastening member or staple. The needle may be hollow or it may be solid at least up to the portion that guides and/or grasps the leg. The diameter of the needle must be such as to readily pass through the opening of the receiver which is going to accept the leg.

When applying a fastener using the instrument of the present invention, two or more tissue portions are approximated in a generally face-to-face relationship. The staple portion of the fastener is disposed on one side of the tissue to be joined and the receiver portion of the fastener is spaced on the opposite side of the tissue to be joined. The instrument having the needle with the guiding and/or grasping means is placed so that the needle passes through the opening in the receiver and penetrates the tissue to guide and/or grasp the free end of the leg of the staple. The needle is removed through the opening in the receiver by reversing its motion. The leg of the staple is caused to follow the guiding and/or grasping needle and the leg of the staple penetrates the tissue. At least a portion of the leg of the staple is passed through the opening of the receiver. The staple leg is guided into the hole in the receiver and aligned therewith. The staple leg is engaged and retained by the receiver and the guiding and/or grasping means removed and the fastener remains holding together the tissue portions.

This instrument may be used for a variety of such types of fasteners. Numerous other features of this novel instrument will be apparent from the following detailed description and accompanying drawings.

In the accompanying drawings, forming part of the specification, like numerals are employed to designate like parts through the same. In the drawings:

Figure 1 is a cross-sectional view of an instrument in accordance with the present invention (in this figure the instrument is in the fully opened position);

Figure 2 is a view in elevation of the instrument depicted in Figure 1 with the instrument in its first closed position;

Figure 3 is a view in elevation of the instrument depicted in Figure 1 with the instrument in its second closed position;

Figure 4 is a view in elevation of the instrument depicted in Figure 1 with the instrument in the open position and with the tissue fastening means in place;

Figure 5 is a cross-sectional view of another instrument of the present invention with the instrument in its initial open position;

Figure 6 is a view in elevation of the instrument depicted in Figure 5 with the instrument in its closed position; and

Figure 7 is a view in elevation of the instrument depicted in Figures 5 and 6 with the instrument in its open position again and with the fastening means in place joining the tissue.

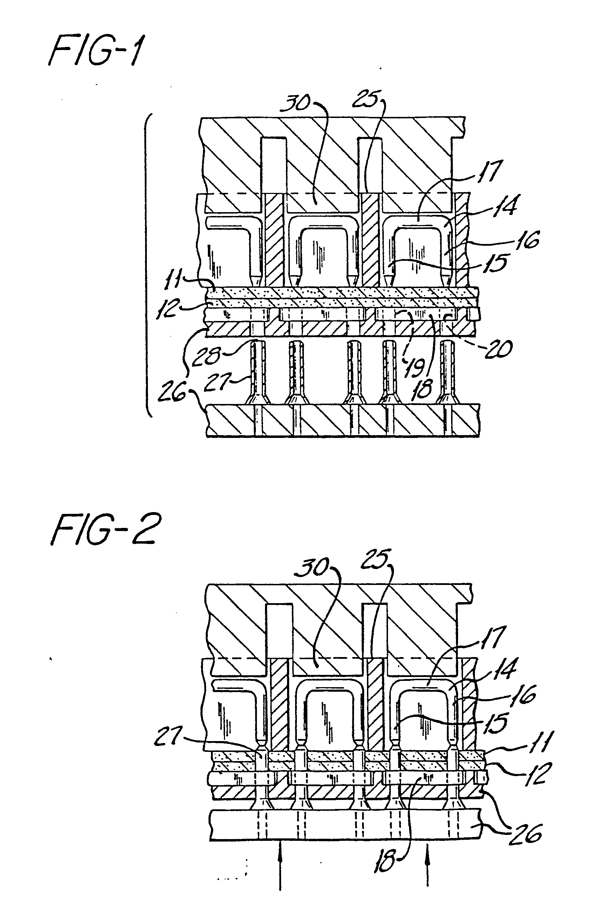

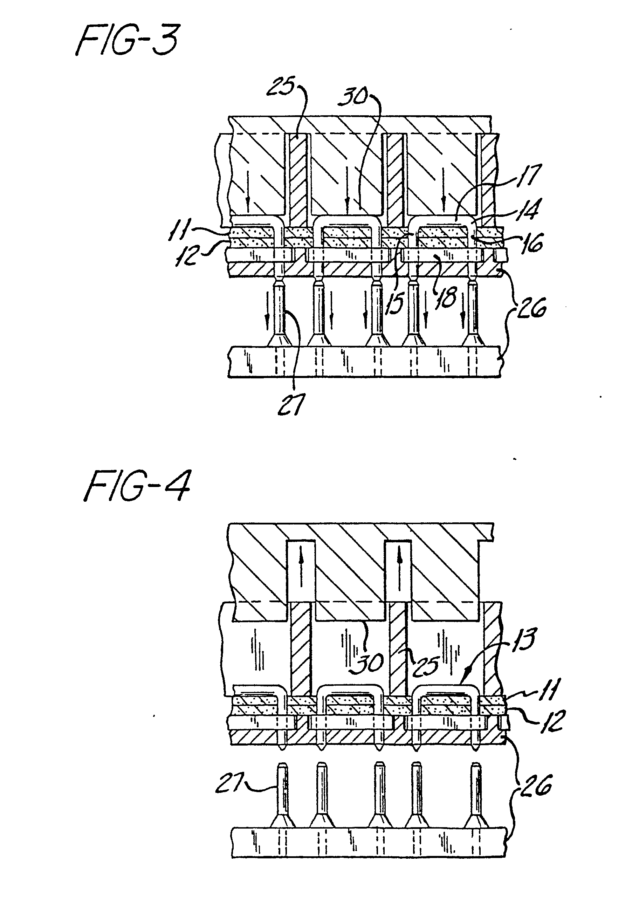

In Figures 1 through 4 there is shown one instrument of the present invention. In this embodiment two pieces of tissue 11 and 12 are being joined by a fastening member comprising a staple 14 having a pair of legs 15 and 16 which are generally parallel and connected by a cross piece 17. The fastening member includes a receiver 18 having a pair of holes or openings 19 and 20 for accepting the legs of the staple and locking therewith. As shown in Figure 1 the staples are carried in one jaw 25 of the instrument and may be held in that jaw either by friction or some type of latch means (not shown for the sake of clarity). This jaw is placed on one side of the two pieces of tissue to be joined. A second jaw 26 of the instrument is placed on the opposite side of the two pieces of tissue to be joined. This jaw carries a plurality of the receivers with the openings in the receivers disposed directly in line with the legs of the staple. The second jaw includes guiding means 27 which is aligned with the openings or holes in the receivers. The free end of each staple leg is tapered and fits into the open end 28 of the guiding means. As shown in Figure 2, the guiding means is inserted through the openings in the receivers through the tissue to be joined so that the open end of the guiding means 28 contacts the free end of each staple leg 15 and 16. At this point, if the staples are being held in the first jaw by a latch, the latch is released and a pusher means 30 is actuated to push the staple 14 and the guiding means 27 back down through the tissue forcing the legs of the staple into the holes of the receiver 18 to be retained thereby. This step is depicted in Figure 3 of the drawings. At this point, the pusher is released and the guiding means may be removed leaving the fastening members in the tissue as depicted in Figure 4.

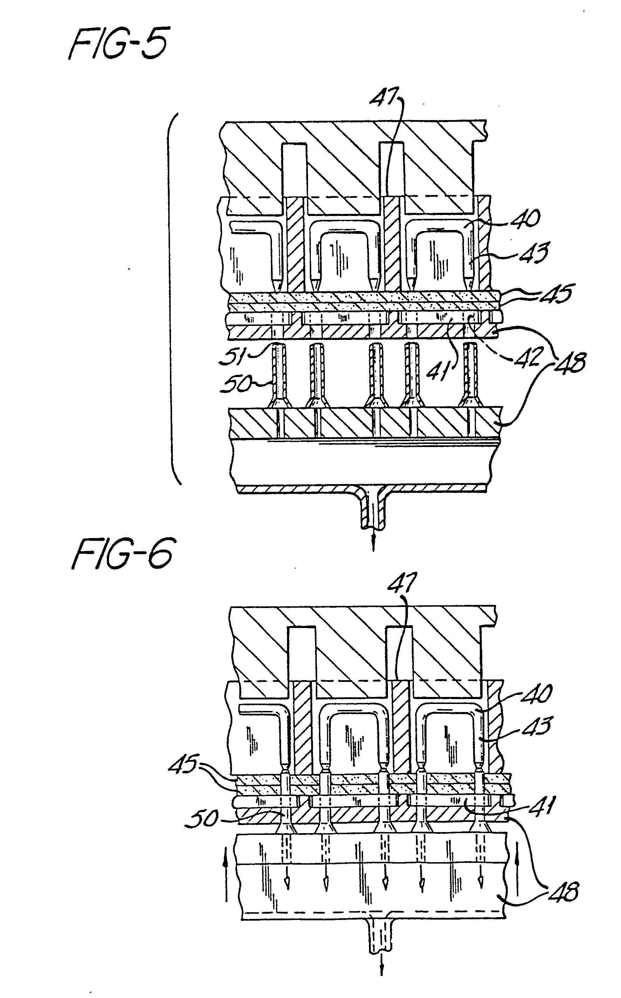

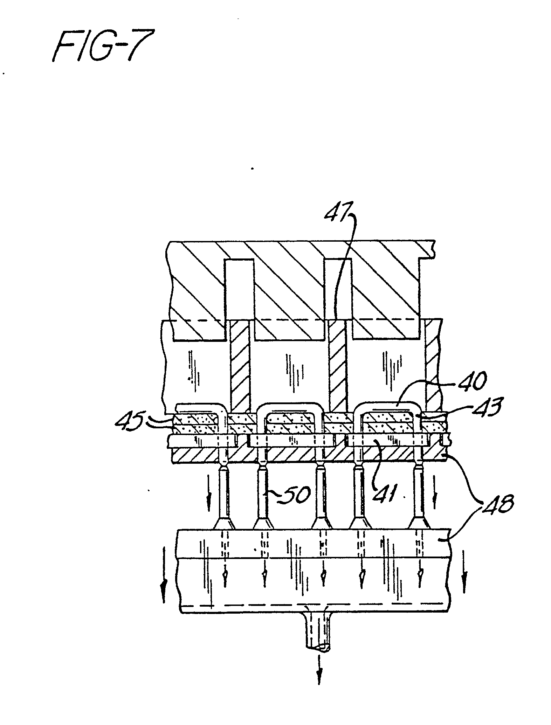

Referring to Figures 5, 6, and 7 there is shown another instrument of the present invention. Again, a two-legged staple 40 and an appropriate receiver 41 having openings 42 for retaining the legs 43 of the staple are disposed in opposite jaws 47 and 48 of the instrument and the opposite jaws placed on the opposite sides of the tissue 40 to be joined. The staples are held in spaces in the jaws by friction as are the receivers. The second jaw 48 includes grasping and guiding means 50 which are hollow. The free ends 51 of the hollow grasping and guiding means are aligned with the openings in the receiver. The free ends are sharpened to aid in tissue penetration. The opposite end of the grasping and guiding means is attached to appropriate suction. In operation, the guiding and grasping means are moved to pass through the openings in the receiver, penetrate the tissue, and contact the free ends of the legs of the staple as shown in Figure 6. At this point, suction is applied to the opposite end of the grasping and guiding means and the means then returned to its original position as shown in Fig. 7. This operation pulls the staple along with the grasping and guiding means through the tissue into engagement with the receivers to be retained thereby. At this point, the suction may be removed and the jaws opened to release the tissue which is joined by the appropriately locked staple and receiver.

Though staples and receivers having a pair of legs and a complementary pair of openings have been described, it should be appreciated that the fasteners may have single legs or multiple legs and the receivers may have single openings or multiple openings or various combinations of the two. The receiver and the fastening member or staple, when in the final position, should be locked in place and this may be accomplished either by the receiver in some manner grasping on to the staple leg such as by an interference fit or by having means on the staple leg to retain the receiver such as barbed areas and the like as are well known in the art.

The staples or fastening members and receivers may be made from various biologically acceptable polymeric materials and these materials may be either of the absorbable type or the non-absorbable type. Suitable absorbable materials are made from the polymers and copolymers of the glycolides, lactides, polydioxanones and the like. Suitable non-absorbable products can be made from the polymers such as polyethylene, polypropylene, polyester, nylon and the like.

The jaws of the instruments for applying fasteners may be joined or they may be separate. The jaws may carry one or virtually any number of fastening members and the other jaw may carry one or any number of receivers for those fastening members. The jaws may either have a shape such as to apply the staples in a straight line or in a circular configuration or in any other configuration or shape as desired. The instrument itself may be disposable or it may be of the reusable variety wherein cartridges carrying both the fastening members and the receivers may be placed in the reusable jaws as is well known in the art. The instrument and fasteners should be sterile and, hence, should be made of materials that are readily sterilizable by the well-known heat, radiation, ethylene-oxide, and similar means for sterilization.

## Claims

1. An instrument, for use in joining mammalian tissue with a two piece fastener comprising a fastening member (14) having a leg (15, 16) for penetrating the tissue to be joined and a receiver (180 having an opening (19, 20) for retaining said fastening member leg, comprising:

means (25) for holding said fastening member (14) on one side of the tissue to be joined with the leg (15, 16) of said member (14) disposed so as to be able to penetrate the tissue to be joined;

means (26) for holding said receiver (18) on the opposite side of the tissue to be joined with the opening (19, 20) in the receiver (18) disposed so as to be able to accept the leg (15, 16) of the fastening member (14);

means (27) for penetrating the tissue through the opening (19, 20) in the receiver (18) and engaging the free end of the leg (15, 16) of the fastening member (14); and

means (30) for moving said leg (15, 16) of the fastening member (14) through the tissue and at least partially into the opening (19, 20) in the receiver (18) to be retained thereby.

2. The instrument of claim 1 wherein the fastening member has a plurality of legs (15, 16), the receiver has a complementary plurality of openings (19, 20), and the penetrating means (27) is arranged to penetrate through each opening and engage each leg.

3. The instrument of claim 1 or claim 2 wherein the means for penetrating the tissue comprises a hollow leg member (27) having its free ends sharpened to aid in the penetration of the tissue for each leg (15, 16) of the fastening member (14).

4. The instrument of claim 3 wherein the means for moving each leg of the fastening member through the tissue is suction applied through each hollow leg member (27) of the penetrating means.

## Patentansprüche

1. Vorrichtung zum Verbinden von Gewebe von Säugetieren mit einer zweistückigen Befestigungsvorrichtung mit einem Befestigungselement (14) mit einem Schenkel (15, 16) zum Durchdringen des zu verbindenden Gewebes und mit einem Aufnehmer (18) mit einer Öffnung (19, 20) zum Halten des Befestigungselement Schenkels, mit

einer Einrichtung (25) zum Festhalten des Befestigungselements (14) auf einer Seite des zu verbindenden Gewebes, wobei der Schenkel (15, 16) des Elementes (14) so angeordnet ist, daß er das zu verbindende Gewebe durchdringen kann, einer Einrichtung (26) zum Festhalten des Aufnehmers (18) auf der gegenüberliegenden Seite des zu verbindenden Gewebes, wobei die Öffnung (19, 20) im Aufnehmer (18) derart angeordnet ist, daß der Schenkel (15, 16) des Befestigungselements (14) einführbar ist,

einer Einrichtung (27) zum Durchsetzen des Gewebes durch die Öffnung (19, 20) in dem Aufnehmer (18) und zum Ergreifen des freien Endes des Schenkels (15, 16) des Befestigungselements (14), und mit

einer Einrichtung (30) zum Bewegen des Schenkels (15, 16) des Befestigungselements (14) durch das Gewebe und zumindest teilweise in die Öffnung (19, 20) im Aufnehmer (18), um dadurch das Befestigungselement festzuhalten.

2. Vorrichtung nach Anspruch 1, dadurch gekennzeichnet, daß das Befestigungselement mehrere Schenkel (15, 16) und der Aufnehmer mehrere dazu komplementäre Öffnungen (19, 20) aufweist und daß die Einrichtung (27) jede Öffnung durchsetzt und mit jedem Schenkel in Eingriff kommt.

3. Vorrichtung nach Anspruch 1 oder 2, dadurch gekennzeichnet, daß die Einrichtung zum Durchsetzen des Gewebes ein hohles Schenkelelement (27) aufweist, dessen freie Enden angeschärft sind, um das Durchdringen des Gewebes durch jeden Schenkel (15, 16) des Befestigungselements (14) zu erleichtern.

4. Vorrichtung nach Anspruch 3, dadurch gekennzeichnet, daß die Einrichtung zum Bewegen jedes Schenkels des Befestigungselements durch das Gewebe mit Unterdruck über jedes hohle Schenkelelement (27) der Durchsetzeinrichtung arbeitet.

## Revendications

1. Instrument à utiliser pour l'assemblage des tissus des mammifères avec un dispositif de serrage en deux parties comprenant un dispositif de serrage (14) ayant une patte (15, 16) destinée à pénétrer dans les tissus à assembler et un récepteur (18) comportant une ouverture (19, 20) pour retenir ladite patte du dispositif de serrage, comprenant:

des moyens (25) pour maintenir ledit dispositif de serrage (14) d'un côté des tissus à assembler avec la patte (15, 16) dudit dispositif (14) placé de façon à être capable de pénétrer dans les tissus à assembler;

des moyens (26) pour maintenir ledit récepteur (18) du côté opposé des tissus à assembler avec l'ouverture (19, 20) du récepteur (18) placé de façon à être capable de recevoir la patte (15, 16) du dispositif de serrage (14);

des moyens (27) pour pénétrer dans les tissus à travers l'ouverture (19, 20) du récepteur (18) et venir en contact avec l'extrémité libre de la patte (15, 16) du dispositif de serrage (14); et

des moyens (30) pour déplacer ladite patte (15, 16) du dispositif de serrage (14) au travers du tissu et au moins partiellement dans l'ouverture (19, 20) du récepteur (18) afin d'y être maintenue.

2. Instrument de la revendication dans lequel le dispositif de serrage comprend une pluralité de pattes (15, 16), le récepteur comprend une pluralité complémentaire d'ouvertures (19, 20), et dans lequel des moyens de pénétration (27) sont agencés pour pénétrer par chaque ouverture et venir en contact avec chaque patte.

3. Instrument selon l'une des revendications 1 ou 2, dans lequel les moyens de pénétration dans les tissus comprennent un élément de patte creuse (27) ayant ses extrémités libres pointues pour faciliter la pénétration dans les tissus de chaque patte (15, 16) du dispositif de serrage (14).

4. Instrument selon la revendication 3, dans lequel les moyens de déplacement de chaque patte de l'élément de serrage au travers des tissus est une aspiration appliqué à travers chaque élément de patte creuse (27) du moyen de pénétration.

0 147 920

*FIG-1*

*FIG-2*

1

*FIG-3*

25
30
17
14
16
11
12
15
18
26
27

*FIG-4*

30
25
13
11
12
27
26

2

*FIG-5*

*FIG-6*

FIG-7